# EUROPÄISCHE PATENTSCHRIFT

(11) **EP 1 490 391 B1**
(45) Veröffentlichungstag und Bekanntmachung des Hinweises auf die Patenterteilung: **21.12.2005**
(21) Anmeldenummer: 03717244.2
(22) Anmeldetag: 27.03.2003
(51) Int. Cl.: C07J 1/00, A61K 31/585, A61P 35/00

(54) **11BETA-LANGKETTIG-SUBSTITUIERTE 19-NOR-17ALPHA-PREGNA-1,3,5(10)-TRIEN-17BETA-OLE MIT EINEM 21,16ALPHA-LAKTONRING**
19-NOR-17ALPHA-PREGNA-1,3,5(10)-TRIEN-17BETA-OLS WITH A 21,16ALPHA-LACTONE RING SUBSTITUTED WITH A LONG CHAIN AT THE 11BETA POSITION
19-NOR-17ALPHA-PREGNA-1,3,5(10)-TRIEN-17BETA-OLS PRESENTANT UN ANNEAU DE LACTONE 21,16ALPHA ET SUBSTITUES PAR UNE CHAINE LONGUE EN POSITION 11BETA

(30) Priorität: 27.03.2002 DE 10214180
(43) Veröffentlichungstag der Anmeldung: 29.12.2004
(73) Patentinhaber: Schering Aktiengesellschaft, 13353 Berlin (DE)
(72) Erfinder: MÜLLER, Gerd, 07745 Jena (DE); HILLISCH, Alexander, 42553 Velbert (DE); HOFFMANN, Jens, 16567 Mühlenbeck (DE); FRITZEMEIER, Karl-Heinrich, 13505 Berlin (DE)
(86) Internationale Anmeldenummer: PCT/EP2003/003226
(87) Internationale Veröffentlichungsnummer: WO 2003/080641

(56) Entgegenhaltungen:
- WO-A-02/26763
- DE-B- 1 255 659
- FR-M- 1 996
- US-A- 3 920 634
- HOBE G ET AL: "SPECIES DIFFERENCES IN BIOTRANSFORMATION OF 17.alpha.-CYANOMETHYLESTR ADIOL 3-METHYL ETHER" STEROIDS: STRUCTURE, FUNCTION, AND REGULATION, ELSEVIER SCIENCE PUBLISHERS, NEW YORK, NY, US, Bd. 36, Nr. 2, August 1980 (1980-08), Seiten 131-147, XP002187197 ISSN: 0039-128X
- DINNER A ET AL: "3 SUBSTITUTED 2 3 DI HYDRO ESTRA-1 3 5 10-TRIENO 16-ALPHA 17-ALPHA-B FURAN-17-BETA-OLS AS POTENTIAL ESTROGENS" JOURNAL OF MEDICINAL CHEMISTRY, AMERICAN CHEMICAL SOCIETY. WASHINGTON, US, Bd. 18, Nr. 3, 1975, Seiten 314-315, XP002187199 ISSN: 0022-2623

## Beschreibung

Die vorliegende Erfindung betrifft neue 11β-langkettig-substituierte 19-Nor-17α-pregna-1,3,5(10)-trien-17β-ole mit einem 21,16α-Laktonring, Verfahren zu deren Herstellung und diese Verbindungen enthaltende pharmazeutische Präparate sowie die Verwendung dieser Verbindungen zur Herstellung von Arzneimitteln.

Estrogene üben ihre physiologische Wirkung über Rezeptorproteine, den Estrogenrezeptoren (ERs) aus. Es handelt sich dabei um kernständige, durch Liganden aktivierbare Transkriptionsfaktoren. Bis vor wenigen Jahren wurde angenommen, dass Estrogene ihre Wirkung über einen einzigen Rezeptor ausüben.

Erst seit 1996 ist bekannt, dass zwei Subtypen des Estrogenrezeptors existieren (ERα und ERβ) (Kuiper et al., Proc. Natl. Acad. Sci. USA 93, 1996, 5925-5930). Beide unterscheiden sich in ihrem Expressionsmuster in unterschiedlichen Geweben. So überwiegt beispielsweise ERβ gegenüber ERα in der Rattenprostata, während ERα im Rattenuterus überwiegt. Im Gehirn wurden Areale identifiziert, in denen jeweils nur einer der beiden ER-Subtypen exprimiert wird (Shugrue et al., Steroids 61, 1996, 678-681; Li et al., Neuroendocrinology 66, 1997, 63-67). In Knochen (Kuiper et al., Frontiers in Neuroendocrinology 1998, 19: 253-286), Blutgefäßen (lafrati et al., Nature Med. 1997, 3: 545-48) und normalem Brustgewebe (Gustafsson und Wamer, J. Steroid Biochem. Mol. Biol. 74, 2000, 245-248) werden sowohl ERα und als auch ERβ exprimiert.

In maligne entartetem Brustgewebe wurde in mehreren unabhängigen Arbeiten eine Aufregulierung der ERα Expression sowie eine verminderte ERβ Expression beobachtet (Leygue et al., Cancer Res. 58, *1998*, 3197-3201; Iwao et al., Int. J. Cancer 88, 2000, 733-736; Lacennec et al., Endocrinology 142, 2001, 4120-4130; Roger et al., Cancer Res. 61, 2001, 2537-2541). ERβ-Knockout-Mäuse (fehlender ERβ) weisen ein abnormales epitheliales Wachstum der Brust und eine Überexpression des Proliferationsmarkers Ki67 auf (Gustafsson and Wamer, 2000).

Auch beim Menschen wurde eine inverse Korrelation zwischen ERβ Expression und Ki67 nachgewiesen (Roger et al., 2001). ERβ fungiert außerdem als Hemmer der ERα transkriptionalen Aktivität und senkt die zelluläre Ansprechbarkeit gegenüber Estradiol (Hall und McDonnell, Endocrinology 140, 1999, 5566-5578). Diese Daten stützen die Hypothese, dass ERβ unter anderem einen schützenden Faktor gegen die durch ERα vermittelte mitogene Aktivität von Estrogenen darstellt ERβ kann daher als ein endogener Gegenspielerdes ERα angesehen werden.

In Patenschriften von Katzenallenbogen et al. (WO 00/19994) und Loozen et al. (WO 00/31112) werden subtypspezifische Estrogenrezeptorliganden, u a ERα selektive Verbindungen, beschrieben.

WO 01/00652 offenbart 11 β-langkettig-substituierte Estratriene der allgemeinen Formel I. 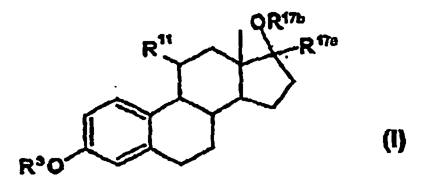 worin R11 ein langkettiger, ein Stickstoff- sowie gegebenenfalls ein Schwefelatom aufwaisender Rest ist, der außerdem endständig mit einer Perfluoralkylgruppe oder einem gegebenenfalls substituierten Arylrest funktionalisiert sein kann. Die Verbindungen verfügen über antiestrogene oder gewebeselektive estrogene Eigenschaften und sind zur Herstellungvon Arzneimitteln geeignet

In der nicht vorveröffentlichten Anmeldung DE 100 48 634 werden 19-Nor-17α-pregna-13,5(10)-trien-17β-ole mit einem 21,16α-Laktonring als selektive Estrogene beschrieben. die im Gegensatz zu klassischen Estrogenen wie Estradiol Präferenz zugunsten des Estrogenrezeptors α aufweisen.

US-A-3920634 beschreibt 2',3'-dihydroestra-1,3,5(10)-trieno[16α,17α-β]furan-17β-olen. die als Arzneimittel zur Behandlung von menopausalen Symptomen und anderen durch Estrogenmangel erzeugte Konditionen verwendet werden. Dinner A. et al. ("2',3'-dihydroestra-1,3,5(10)-trieno[16α,17α-β]furan-17β-als as Potential Estrogen", Journal of Medicinal Chemistry. American Chemical Society, 1975, Bd.18, Nr.3, Seiten 314-315) bezieht sich auf die in US-A-3920634 gezeigten Verbindungen und beschreibt weiter die estrogene Wirkung in einem Verfahren zum Messen der Uterus-Gewichtszunahme von behandelten noch nicht voll entwickelten Mäusen, im Vergleich zu einer unbehandelten Kontrollgruppe.
Die obengenannten Verbindungen unterscheiden sich durch die Substituenten an den Positionen 11, 16 und 17. US-A-3920634 und Dinner at al. beschreiben keine selektive Bindungsaffinität für ERα Ober ERβ.
DE-B-1255659 und FR-B-1996M beschreiben Steroidlactone, die ein Wasserstoffatom an 11. Position und eine pentazyklische Struktur enthalten.
FR-B-1996M insbesondere beschreibt eine anti-androgene Wirkung, die durch eine Gewichtsabnahme der Prostata gemessen wird. Diese anti-androgene Wirkung kann zur Behandlung von spezifischen endokrinen Karzinome wie zum Beispiel Prostatakarzinoma und zur Behandlung von Endokrinmangel Verwendet werden.
Hobe G. et al. ("Species Differences in Biotransformation of 17a-Cyanomethylestradiol 3-Methyl Ether", Steroids, 1980, Bd. 36, Nr. 2, Seite 131-147) untersucht Artendifferenzen in der Biotrasformation von steroiden Derivaten durch den Vergleich der Muster der Metaboliten in Urin and Gallenflüssigkeit.
Es sind die Muster der Metaboliten in Menschen, Pavian, Beagle Hunden, Minischweinen und Ratten untersucht worden. Außerdem, ist die Bedeutung der 17α-Position der steroiden Derivaten für den Effekt und Wirkungsweise der genannten Verbindungen deutlich aufgezeigt worden.

Die Aufgabe der vorliegenden Erfindung besteht darin, neue Verbindungen zur Verfügung zu stellen, die *in vitro* eine Dissoziation hinsichtlich ihrer Bindung an Estrogenrezeptorpräparationen von Rattenprostata und Rattenuterus aufweisen und *in vivo* über die präferenzielle Antagonisierung des ERa, ohne die positiven Eigenschaften des ERβ zu unterbinden, eine antiproliferative Wirkung. Dies schließt auch eine präferenzielle Unterdrückung der Expression von ERα ohne Reduktion der ERβ Expression mit ein.

Die Aufgabe wird gemäß vorliegender Erfindung durch die Bereitstellung von neuartigen 19-Nor-17α-pregna-1,3,5(10)-trien-17β-olen mit einem 21,16α-Laktonring mit einem langkettigen Substituenten in 11β-Stellung der allgemeinen Formel II 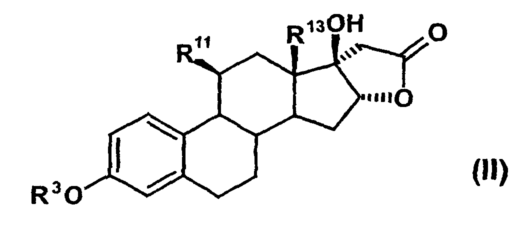 worin
- R³: ein Wasserstoffatom, eine C₁₋₄-Alkyl, C₂₋₆-Acyl- oder Tri(C₁₋₄-alkyl)silylgruppe oder eine Gruppe R¹⁸SO₂-,
wobei
R¹⁸ eine Gruppe R¹⁹R²⁰N-,
worin
R¹⁹ und R²⁰ unabhängig voneinander ein Wasserstoffatom, ein C₁₋₅-Alkylrest, eine Gruppe C(O)R²¹ sind und
worin
R²¹ einen geradkettigen oder verzweigten Kohlenwasserstoffrest mit bis zu 6 Kohlenstoffatomen, einen C₃₋₇-Cycloalkylrest, einen Arylrest, der gegebenenfalls substituiert sein kann, einen Aralkyl- oder einen Alkylarylrest darstellt,
und
- R¹¹: einen geradkettigen Alkylrest mit 6 bis 17 Kohlenstoffatomen
und
- R¹³: eine Methyl- oder Ethylgruppe
bedeuten.

Ein weiterer Gegenstand der vorliegenden Erfindung betrifft Arzneimittel, die eine Verbindung der allgemeinen Formel II oder deren pharmazeutisch annehmbare Additionssalze mit organischen oder anorganischen Säuren enthalten.

Wenn nicht näher definiert, handelt es sich im Sinne der vorliegenden Erfindung bei einem Arylrest, der gegebenenfalls substituiert sein kann, um einen Phenyl-, 1- oder 2-Naphthylrest, wobei der Phenylrest bevorzugt ist. Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl, der 2- oder 3-Thienyl, der 2- oder 3-Pyrrolyl-, der 2-, 4- oder 5-Imidazolyl-, der Pyrazinyl-, der 2-, 4-oder 5-Pyrimidinyl- oder 3- oder 4-Pyridazinylrest.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, erwähnt.

Für den Substituenten R¹¹ seien als Vertreter für geradkettige Alkylketten mit 6 bis 17 Kohlenstoffatomen beispielsweise Hexyl, Heptyl, Decyl, Dodecyl genannt.

Als Vertreter für Alkylreste bzw. gerad- oder verzweigtkettige Alkylgruppen mit bis zu 12 Kohlenstoffatomen im Sinne von R³, R¹⁹ und R²⁰ bzw. R²¹ sind beispielsweise Methyl, Ethyl, Propyl, Isopropyl, Butyl, Isobutyl, tert.-Butyl, Pentyl, Isopentyl, Neopentyl, Hexyl, Heptyl, Decyl zu nennen. Methyl, Ethyl, Propyl und Isopropyl sind bevorzugt.

C₂₋₆-Acylreste bedeuten beispielsweise Acetyl, Propionyl, Butyryl, Valeroyl, Isovaleroyl, Pivaloyl, Hexanoyl.

Vertreter für die vorstehend genannte C₃₋₇-Cycloalkylgruppe können beispielsweise Cyclopropyl, Cyclobutyl, Cyclopentyl, Cyclohexyl sein

Die Hydroxylgruppe am C-Atom 3 kann mit einer aliphatischen, gerad- oder verzweigtkettigen, gesättigten oder ungesättigten C₂₋₆-Carbonsäure verestert sein. Als derartige Carbonsäuren zur Veresterung kommen beispielsweise in Betracht: Essigsäure, Propionsäure, Buttersäure, Isobuttersäure, Valeriansäure, Isovaleriansäure oder Pivalinsäure.

Als Beispiele für eine Tri(C₁₋₄-alkyl)gruppe seien eine Trimethylsilylgruppe und eine tert-Butyldimethylgruppe genannt.

Erfindungsgemäß bevorzugt sind Verbindungen der allgemeinen Formel II 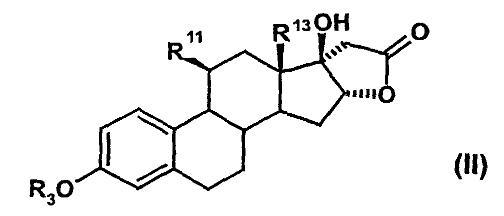 worin
R³ ein Wasserstoffatom oder eine Methylgruppe sowie R¹³ eine Methylgruppe bedeuten und R¹¹ aus der Gruppe der folgenden Seitenketten ausgewählt sein kann: Hexyl, Octyl, Decyl, Dodecyl.

Besonders bevorzugte 11β-langkettig substituierte 19-Nor-17α-pregna-1,3,5(10)-triene mit einem 21,16α-Laktonring sind beispielsweise:
3,17β-Dihydroxy-11β-hexyl-19-nor-17α-pregna-1,3,5(10)-trien-21,16α-lakton
3,17β-Dihydroxy-11β-octyl-19-nor-17α-pregna-1, 3,5(10)-trien-21,16α-lakton
3,17β-Dihydroxy-11β-decyl-19-nor-17α-pregna-1,3,5(10)-trien-21,16α-lakton
3,17β-Dihydroxy-11β-dodecyl-19-nor-17α-pregna-1,3,5(10)trien-21,16α-lakton

Für die Bildung von pharmazeutisch verträglichen Salzen der erfindungsgemäßen Verbindungen der allgemeinen Formel II kommen als anorganische Säuren unter anderem Chlonwasserstoffsäure, Bromwasserstoffsäure, Schwefelsäure und Phosphorsäure, sowie als organische Säuren unter anderem Essigsäure, Propionsäure, Maleinsäure, Fumarsäure, Bernsteinsäure, Benzoesäure, Ascorbinsäure, Oxalsäure, Salicyläure, Weinsäure, Zitronensäure, Milchsäure, Äpfelsäure, Mandelsäure, Zimtsäure und Methansulfonsäure in Betracht.

Die erfindungsgemäßen Substanzen gemäß allgemeiner Formel II stellen Verbindungen dar, die sich gegenüber den aus dem Stand der Technik bekannten Verbindungen mit langkettigen 11β-Seitenketten durch ein neues Strukturelement, einen 21,16α-Laktonring unterscheiden. Es wurde gefunden, dass erfindungsgemäße 11β-substituierte 19-Nor-17α-pregna-1,3,5(10)-trien-17β-ole mit einem 21,16α-Laktonring selektiv am ERα in überraschender Weise eine antagonistische Wirkung zeigen. Mit den erfindungsgemäßen Substanzen gelingt es präferenziell den ERα zu antagonisieren, ohne die positiven Eigenschaften des ERβ zu unterbinden. Dies schließt auch eine präferenzielle Unterdrückung der Expression von ERα ohne Reduktion der ERβ Expression mit ein.

### Biologische Charakterisierung der erfindungsgemäßen Verbindungen

Die erfindungsgemäßen Substanzen der allgemeinen Formel II wurden in verschiedenen Modellen getestet. Die erfindungsgemäßen Substanzen üben neben dem Brustgewebe auch in anderen hormonmodulierten Tumoren über die selektive Hemmung des ERα eine antiproliferative Wirkung aus.

### Estrogenrezeptorbindungsstudien

### Methodik

Die Bindungsaffinität der neuen selektiven Antiestrogene wurde in Kompetitionsexperimenten unter Verwendung von 3H-Estradiol als Ligand an Estrogenrezeptor präparationen von Rattenprostata und Rattenuterus getestet. Die Präparation des Prostatacytosols und der Estrogenrezeptortest mit dem Prostatacytosol wurde, wie in der Literatur beschrieben, durchgeführt (Testas et al., Endocrinology 109, 1981, 1287-1289).

Die Präparation von Rattenuteruscytosol sowie der Rezeptortest mit dem Estrogenrezeptor-haltigen Cytosol wurde prinzipiell nach Stack und Gorski (Stack, Gorski, Endocrinology 117, 1985, 2024-2032) mit einigen Modifikationen (Fuhrmann et al., Contraception 51, 1995, 45-52) durchgeführt.

Die erfindungsgemäßen Substanzen, wie beispielsweise 3,17β-Dihydroxy-11β-hexyl-19-nor-17α-pregna-1,3,5(10)-trien-21,16α-lakton (Verbindung 1) weisen eine höhere Bindungsaffinität zum Estrogenrezeptor aus dem Rattenuterus als zum Estrogenrezeptor aus der Rattenprostata auf (siehe Tabelle 1).

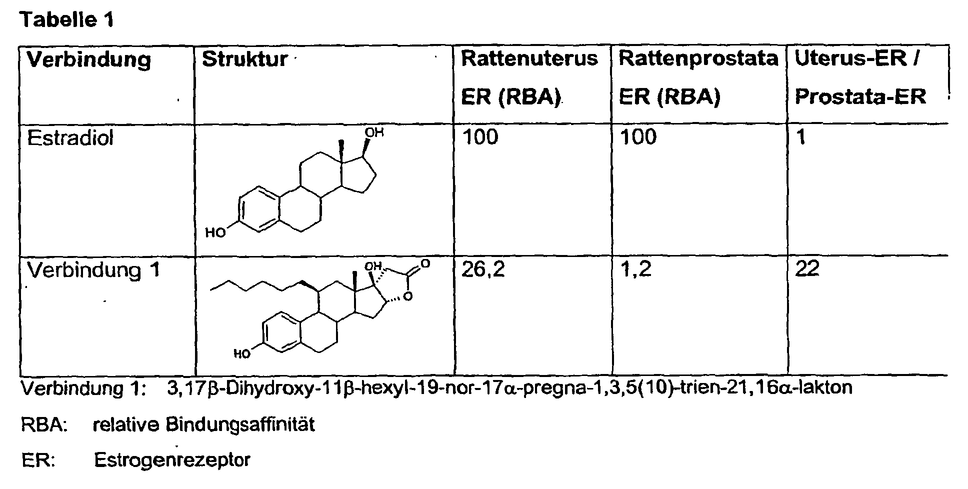

Dabei wird davon ausgegangen, dass in der Rattenprostata ERβ gegenüber ERα, dagegen im Rattenuterus ERα gegenüber ERβ überwiegt. Tabelle 2 zeigt, dass das Verhältnis der Bindung an Prostata- und Uterusrezeptor qualitativ dem Quotienten der relativen Bindungsaffinität (RBA) an humanen ERβ und ERα der Ratte entspricht (Kuiper et al., Endocrinology 138, 1996, 863-870).

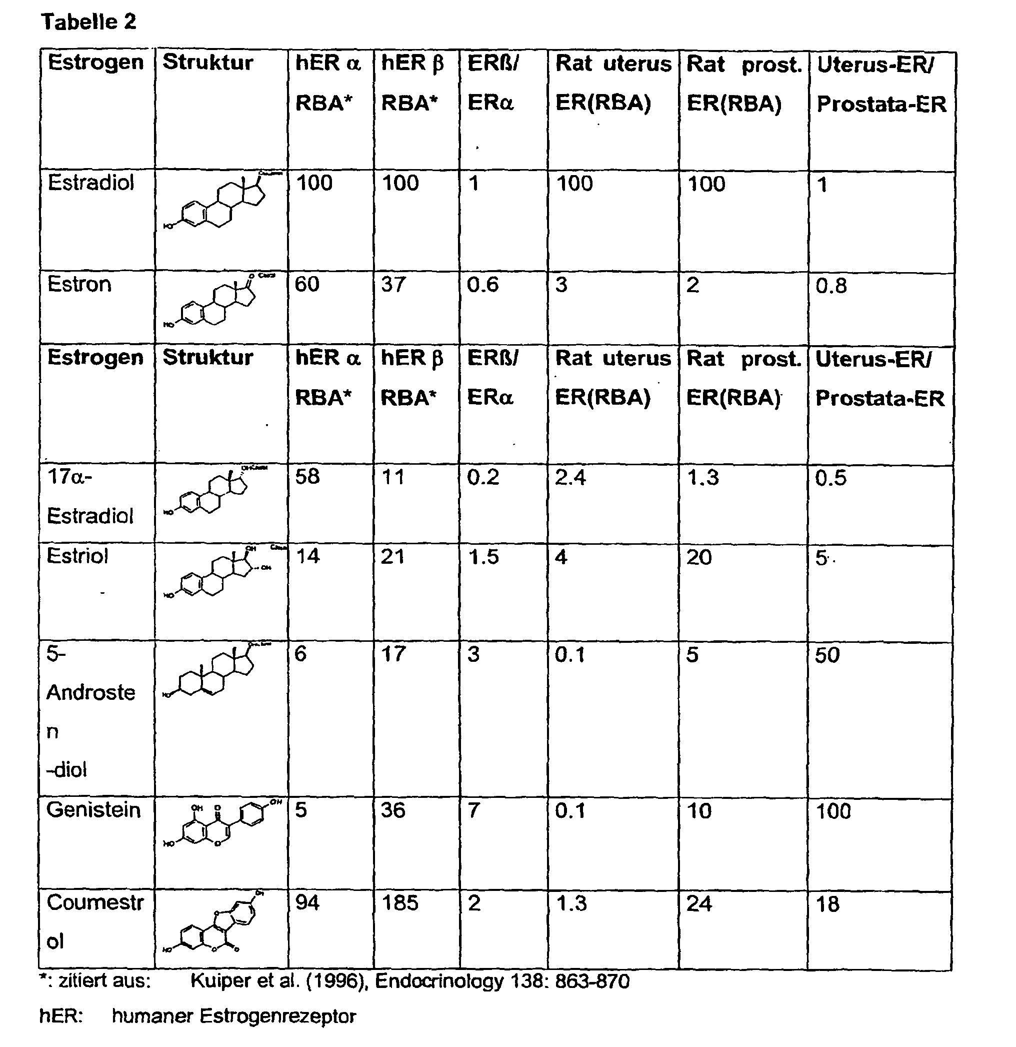

### Zellproliferationsassay

Die Mammakarzinomzelllinie MCF-7 ist eine hormonabhängige humane Zelllinie. Das Wachstum dieser ER-positiven Zellen wird durch Estradiol stimuliert und ist mit partialagonistischen und reinen Antiestrogenen antagonisierbar (Lippman et al. 1976, Wakelin et al. 1991). Die erfindungsgemäßen Substanzen, beispielsweise 3,17β-Dihydroxy-11β-hexyl-19-nor-17α-pregna-1,3,5(10)-trien-21,16α-lakton (Verbindung 1), üben auf die durch Estradiol modulierten Tumore eine antiproliferative Wirkung über die selektive Hemmung des ERα aus.

### Material und Methoden

Die gut charakterisierte Mammakarzinomzellinie MCF-7 wurde aus dem Pleuraexsudat einer 69-jährigen Patientin mit disseminiertem Mammakarzinom etabliert (Soule et al. 1973).
Die Zellen werden in RPMI Medium 1640 w/o Phenolrot, 1% L-Glutamin, 200mMol Insulin/ml und 10 % FCS bzw. CCS im Brutschrank bei 37°C und wasserdampfgesättigter Atmosphäre mit 5 % CO₂ kultiviert.
Alle drei bis vier Tage findet ein Mediumwechsel statt. Zellen, die in der Zellkulturflasche zu 80-90 % konfluent wachsen, werden passagiert.

In den Versuchen wurden die Prüfsubstanzen auf ihre durch Estradiol stimulierten proliferationshemmenden Eigenschaften untersucht.

Für die Versuche erfolgt die Aussaat einer definierten Zellzahl in einer 96-Lochplatte in 200 µl Medium/Loch (5000 Zellen/ Loch). Zwei Spalten werden als Leerwerte nur mit Medium gefüllt. 24 Stunden später, nach erfolgter Adhäsion der Zellen, wird das Medium unter Beifügung der zu prüfenden Substanzen gewechselt. Die Testsubstanzen, als Stammlösung in Ethanol absolut (EtOH) gelöst, werden in aufsteigenden Konzentrationen zugegeben. Neben einer Lösungsmittel-Kontrolle wird auch eine Estradiol-Kontrolle eingesetzt. Die Versuchsdauer beträgt 7 Tage, innerhalb derer ein Mediumwechsel nach drei Tagen durchgeführt wird. Die Proliferation wird mit dem Kristallviolett-Assay bestimmt. Diese Methode beruht auf der Anfärbung der DNA im Zellkern durch Kristallviolett (N-Hexamethylpararosanilin) (Wakelin et al. 1981). Die Intensität der Blaufärbung, die abhängig von der Menge des an die DNA gebundenen Kristallvioletts ist, kann durch spektralphotometrische Messungen quantifiziert werden. Die Extinktionswerte entsprechen der DNA-Menge und damit der Zellkernmenge. Sie werden als indirekte Parameter der Zellzahl gleichgesetzt (Gillies et al. 1986).

Die Zellen werden bei Versuchsende mit 25 µl Glutardialdehydlösung (11%) pro Loch für 20 Minuten bei Raumtemperatur durch leichtes Schütteln fixiert. Die Lochplatte wird ausgeschlagen, dreimal unter fließendem, entsalzten Wasser (VE-Wasser) gewaschen und luftgetrocknet. Nach Zugabe von 100 µl Kristallviolett (0,1%, pH 4,5) pro Loch auf die fixierten Zellen wird 20 Minuten bei Raumtemperatur geschüttelt. Nach erneutem Waschen und Lufttrocknen werden 100 µl Essigsäure (10%) je Loch zugegeben, kurz geschüttelt und die Extinktion bei 595 nm am Spektralphotometer gemessen (Kueng et al. 1989).

### Ergebnisse

Wie aus Schema 1 ersichtlich, hemmt 3,17β-Dihydroxy-11β-hexyl-19-nor-17α-pregna-1,3,5(10)-trien-21,16α-lakton (Verbindung 1) das Wachstum von MCF-7 Mammakarzinomzellen. Es besteht eine Dosis-Wirkungsbeziehung. Bei einer Konzentration von 1x10⁻⁵ M wird die durch Estradiol stimulierte Proliferation der MCF-7 Mammakarzinomzellen zu 100% gehemmt.

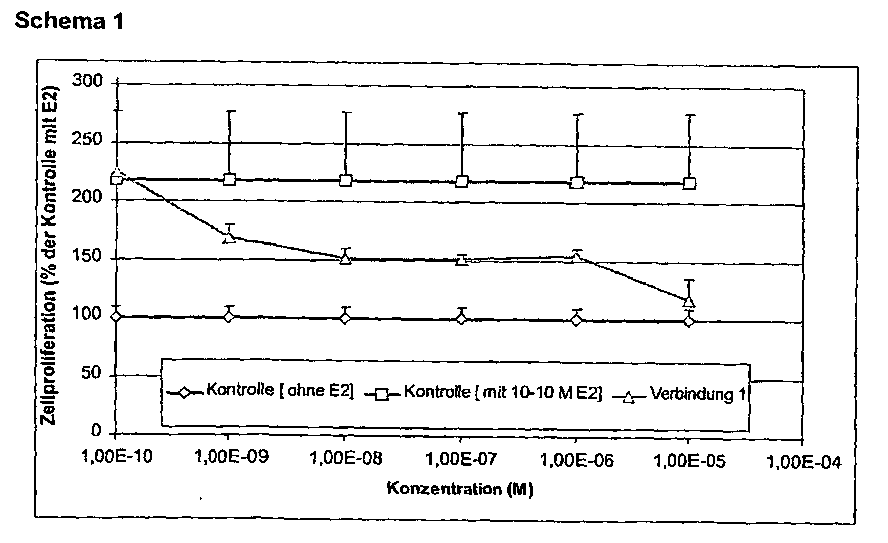

### Pharmazeutische Präparate und Indikationen

Die Verbindungen der allgemeinen Formel II stellen Verbindungen mit antiestrogener Wirksamkeit nach peroraler oder parenteraler Applikation dar.

Darüber hinaus handelt es sich bei den erfindungsgemäßen Verbindungen um reine Antiestrogene.

Die vorliegende Erfindung umfasst die neuartigen Substanzen als pharmazeutische Wirkstoffe, deren Herstellung, ihre therapeutische Anwendung und die pharmazeutischen Darreichungsformen, die die neuen Substanzen enthalten. Bei den chemischen Verbindungen handelt es sich um neue steroidale ERα-selektive Antagonisten.

Die im vorliegenden Patent beschriebenen neuen selektiven ERα-Antagonisten können als Einzelkomponente oder in Kombination insbesondere mit Estrogenen oder Antigestagenen in pharmazeutischen Zubereitungen eingesetzt werden. Die neuartigen selektiven ERα-Antagonisten eignen sich sowohl zur Behandlung von estrogen-abhängigen Erkrankungen, wie zum Beispiel Endometriose, von Mammakarzinomen, Endometriumskarzinomen, antiovulatorischer Infertilität als auch zur Behandlung von Prostatakarzinomen, Prostatahyperplasien, Melanomen sowie Lungenkarzinomen.

Die Verbindungen der allgemeinen Formel II können als Komponente in den in der EP 346014 B1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.

Die Verbindungen der allgemeinen Formel II können gemeinsam mit Antigestagenen (kompetitive Progesteronantagonisten) zur Behandlung hormonabhängiger Tumoren verwendet werden (EP 310 542 A).

Weitere Indikationen, in denen Verbindungen der allgemeinen Formel II zum Einsatz kommen können, sind der männliche Haarausfall, eine diffuse Alopecie, eine durch eine Chemotherapie hervorgerufene Alopecie sowie Hirsutismus (H.-S. Oh, R. C. Smart, Proc. Natl. Acad. Sci. USA, 93, 1996, 12525-12530).

Ferner kann man die Verbindungen der allgemeinen Formel II zur Herstellung pharmazeutischer Zusammensetzungen für die männliche und weibliche Fertilitätskontrolle einsetzen.

Die erfindungsgemäßen Verbindungen der allgemeinen Formel II und deren Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff mindestens einen oder mehrere der erfindungsgemäßen Verbindungen der allgemeinen Formel II oder deren Säureadditionssalze, gegebenenfalls in Kombination mit anderen pharmakologisch wirksamen Stoffen bzw. pharmazeutischen Hilfsstoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen bzw. angegeben sind: Ullmann's Enzyklopädie der technischen Chemie, 4, 1953, 1-39; J. Pharm. Sciences, 52, 1963, 918 ff; H. v. Czetsch-Lindenwald, Hilfsstoffe für Pharmazie und angrenzende Gebiete; Pharm. Ind. 2, 1961, 72 ff; Dr. H. P, Fiedler, Lexikon der Hilfsstoffe für Pharmazie, Kosmetik und angrenzende Gebiete Cantor KG. Aulendorf in Württemberg 1971.

Die erfindungsgemäßen Verbindungen der allgemeinen Formel II können oral oder parenteral, z.B. intraperitoneal, intramuskulär, subkutan und perkutan verabreicht werden. Die Verbindungen können auch in das Gewebe implantiert werden.

### Dosierung

Die zu verabreichende Menge der Verbindungen schwankt innerhalb eines weiten Bereiches und kann jede wirksame Menge abdecken. In Abhängigkeit des zu behandelnden Zustandes und der Art der Verabreichung kann die Menge der verabreichten Verbindung 0,1-25 mg/ kg Körpergewicht, vorzugsweise 0,5-5 mg/ kg Körpergewicht betragen. Beim Menschen entspricht das einer täglichen Dosis von 5 bis 1250 mg. Die bevorzugte tägliche Dosierung beim Menschen ist 50 bis 200 mg.

Zur oralen Verabreichungen kommen Tabletten, Filmtabletten, Dragees, Kapseln, Pillen, Pulver, Lösungen oder Suspensionen oder auch Depotformen infrage. Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel II können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel II enthaltende Kapseln können beispielsweise hergestellt werden, indem man die Verbindung der allgemeinen Formel II mit einem inerten Träger wie Milchzucker oder Sorbit mischt und in Gelatinekapseln einkapselt.

Um eine bessere Bioverfügbarkeit des Wirkstoffes zu erreichen, können die Verbindungen der allgemeinen Formel II auch als Cyclodextrinclathrate formuliert werden. Hierzu werden die Verbindungen mit α-, β- oder γ-Cyclodextrin oder Derivaten von diesen umgesetzt (PCT/EP 95/02656).

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnussöl, Baumwollsamenöl, Sojabohnenöl, Rizinusöl und Sesamöl.

Die Verbindungen der allgemeinen Formel II können auch in Form einer Lösung formuliert werden, die für die orale Verabreichung bestimmt ist und die neben der aktiven Verbindung der allgemeinen Formel II enthält
a) ein pharmazeutisch verträgliches Öl und/ oder
b) eine pharmazeutisch verträgliche lipophile oberflächenaktive Substanz und/ oder
c) eine pharmazeutisch verträgliche hydrophile oberflächenaktive Substanz und/oder
d) ein pharmazeutisch verträgliches, mit Wasser mischbares Lösungsmittel.
Hierzu wird außerdem auf die WO 97/21440 verwiesen.

Die Verbindungen lassen sich auch in Form einer Depotinjektion oder eines Implantatpräpats anwenden, die so formuliert sein können, dass eine verzögerte Freigabe des Wirkstoffes ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel II beladenen Intravaginal- (z. B. Vaginalringe) oder Intrauterinsysteme (z.B. Pessare, Spiralen) eignen sich verschiedene Polymere wie zum Beispiel Silikonpolymere, Ethylenvinylacetat, Polyethylen oder Polypropylen.

Die erfindungsgemäßen Verbindungen der allgemeinen Formel II lassen sich wie nachstehend beschrieben herstellen.

Die Herstellung der 11β-substituierten 19-Nor-17α-pregna-1,3,5(10)-trien-17β-ole mit einem 21,16α-Laktonring kann in einem Einstufenverfahren aus den entsprechenden 17-Oxo-verbindungen bzw. den 17α-cyanomethylierten Estra-1,3,5(10)-trienderivaten erfolgen (nicht vorveröffentlichte DE 100 48 634). Die Ausbildung des Iminoethers . und damit auch des Laktons ist an das Vorhandensein eines 17α-Cyanomethylsubstituenten gebunden.

Ausgangsstoffe für die Synthese der 11β-alkylsubstituierten 17β-Hydroxy-19-nor-17α-pregna-1,3,5(10)-trien-21,16α-laktone sind Verbindungen der allgemeinen Formel III 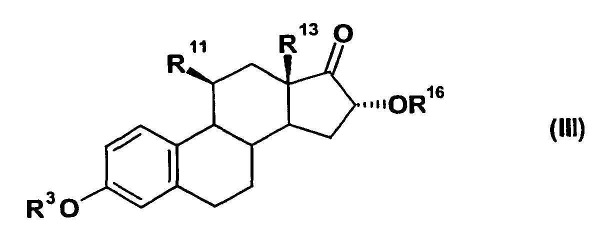 worin
- R³: eine C₁₋₄-Alkyl, C₂₋₆-Acyl- oder Tri(C₁₋₄-alkyl)silylgruppe,
- R¹¹: einen geradkettiger Alkylrest mit 6 bis 17 Kohlenstoffatomen,
- R¹³: Methyl- oder Ethylgruppe,
- R¹⁶: Acetyl- oder Trimethylsilylgruppe
bedeuten.

Die 11β-Alkylsubstitution erfolgt durch regio- und stereoselektive Synthese wie in der Literatur beschrieben. Das elektrophile Estradiolderivat, beispielsweise 5α,10α-Epoxy-estr-9(11)en-Derivat, wird aus einem in 3-Stellung geschützten Estra-5(10),9(11)-dien-17β-ol hergestellt (Teutsch et al. US 4 447 424; Faraj et al. J. Chem. Soc., Perkin Trans 1, 1990, 3045-3048). Reaktion mit Grignardreagenzien R¹¹MgX (X = Halogen wie Brom, Chlor) in Gegenwart katalytischer Mengen von Kupfer(I)-chlorid führt zum 11β-alkylsubstituierten 5α-Hydroxy-9(10)en-derivat (Belanger et al., Steroids 37, 1981, 361-382). Unter Zusatz von Säuren erfolgt simultan die Deketalisierung und Dehydratisierung der 11β-alkylsubstituierten Verbindung, die in eine anschließende Isomerisierungsreaktion mit Wasserstoff unter Zusatz eines Katalysators eingesetzt wird. Das so erhaltene 11β-alkylsubstituierte Estra-1,3,5(10)-trien-17-on-derivat wird in 3-Stellung geschützt und anschließend in herkömmlicher Weise in eine 16α-Bromverbindung überführt. Durch alkalische Hydrolyse und Schutz der erhaltenen 16α-Hydroxyfunktion erhält man Verbindungen der allgemeinen Formel III.

Alternativ dazu kann die Einführung der 16-Hydroxyfunktion durch Umsetzung von 17-Silyl- oder 17-Acylenolethem mit Persäuren und anschließender Hydrolyse durchgeführt werden.

Durch Reaktion der Verbindungen der allgemeinen Formel III mit *in situ* hergestelltem Lithiumacetonitril entsteht intermediär ein 17α-Cyanomethyl-16α-Hydroxylat. Durch Addition des 16α-Alkoholates an die Nitrilgruppe und anschließende Hydrolyse des gebildeten Iminoethers wird das Lakton gebildet.

Durch die Verwendung von Verbindungen gemäss der allgemeinen Formel III, worin R¹⁶ Trimethylsilyl oder Acetyl bedeutet, kann in einem Eintopfverfahren ein Anteil von ca. 60 % 17α-cyanomethyliertem Produkt *in situ* zum 21,16α-Lakton umgesetzt werden.

Die Verseifung von Estergruppierungen sowie Veretherung und/oder Veresterung freier Hydroxylgruppen erfolgt jeweils nach etablierten Verfahren der organischen Chemie.

Die erfindungsgemäßen Sulfamate sind in an sich bekannter Weise aus den entsprechenden Hydroxy-Steroiden durch Veresterung mit Sulfamoylchloriden in Gegenwart einer Base zugänglich (Z. Chem. 15, 270-272 (1975); Steroids **61,** 710 - 717 (1996)).

Nachfolgende Acylierung der Sulfamidgruppe führt zu den erfindungsgemäßen (N-Acyl)sulfamaten (vgl. DE 195 40 233 A1).

Die Säureadditionssalze der Verbindungen der allgemeinen Formel II lassen sich ebenfalls nach gängigen Verfahren aus den freien Säuren der Verbindungen der allgemeinen Formel II herstellen.

Die nachfolgenden Beispiele dienen der näheren Erläuterung der Erfindung, ohne sie darauf einzuschränken:

### Allgemeines Herstellungsverfahren

8 ml (20 mmol) n-Butyllithiumlösung (2,5 M in Toluol) werden in einem inertisierten Reaktionsgefäß unter Rühren auf -25°C bis -35°C abkühlt. Anschließend verdünnt man die Lösung durch Zugabe von 8 ml Tetrahydrofuran unter Kühlen und setzt mit 1,15 ml (22 mmol) Acetonitril im genannten Temperaturbereich um. Es entsteht eine weiße bis gelbliche Suspension an Lithiumacetonitril.
Zu dieser Suspension gibt man eine Lösung von 2,5 mmol des Steroids der allgemeinen Formel III (z.B. 1,14 g 11β-Hexyl-17-oxo-estra-1,3,5(10)-trien-3,16α-diyldiacetat) in 8 ml Tetrahydrofuran unter Einhalten der Reaktionstemperatur von -25°C bis -35°C.
Nach einer Stunde Reaktionszeit im genannten Temperaturbereich wird der Ansatz mit Wasser versetzt, mit verdünnter Salzsäure neutralisiert, das Tetrahydrofuran abdestilliert und das Rohproduktgemisch durch Extraktion mit Essigester isoliert.
Durch Chromatografie über Kieselgel 60 mit einem Eluentengemisch von Chloroform/ n-Hexan/ Methanol (45/45/10) kann das Produkt abgetrennt und isoliert werden.

### Beispiel 1

Aus 2,5 mmol (1,14 g) 11β-Hexyl-17-oxo-estra-1,3,5(10)-trien-3,16α-diyl-diacetat werden 3,17β-Dihydroxy-11β-hexyl-19-nor-17α-pregna-1,3,5(10)-trien-21,16α-lakton 546 mg (53 % d.Th.) erhalten.

Analog werden außerdem folgende Verbindungen erhalten:
3,17β-Dihydroxy-11β-octyl-19-nor-17α-pregna-1,3,5(10)-trien-21,16α-lakton
3,17β-Dihydroxy-11β-decyl-19-nor-17α-pregna-1,3,5(10)-trien-21,16α-lakton
3,17β-Dihydroxy-11β-dodecyl-19-nor-17α-pregna-1,3,5(10)-trien-21,16α-lakton

## Patentansprüche

1. 19-Nor-17α-pregna-1,3,5(10)-triene mit einem 21,16α-Laktonring der allgemeinen Formel (II) 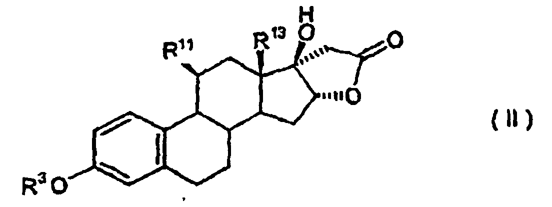 worin
R³ ein Wasserstoffatom, eine C₁₋₄-Alkyl, C₂₋₆-Acyl- oder Tri(C₁₋₄-alkyl)silylgruppe oder eine Gruppe R¹⁸SO₂-,
wobei
R¹⁸eine Gruppe R¹⁹R²⁰N-,
worin
R¹⁹ und R¹⁸ unabhängig voneinander ein Wasserstoffatom, ein C₁₋₅-Alkylrest, eine Gruppe C(O)R²¹ sind und
worin
R²¹ einen geradkettigen oder verzweigten Kohlenwasserstoffrest mit bis zu 6 Kohlenstoffatomen, einen C₃₋₇-Cycloalkylrest, einen Arylrest, der gegebenenfalls substituiert sein kann, einen Aralkyl- oder einen Alkylarylrest bedeutet, und
R¹¹ einen geradkettigen Alkylrest mit 6 bis 17 Kohlenstoffatomen
und
R¹³ eine Methyl- oder Ethylgruppe
bedeuten.

2. 11β-substituierte 19-Nor-17α-pregna-1,3,5(10)-tiene mit einem 21,16α-Laktonring nach Anspruch 1, **dadurch gekennzeichnet, dass** die Verbindungen selektive Estrogenrezeptor α-Antagonisten darstellen.

3. 11β-substituierte 19-Nor-17α-pregna-1,3,5(10)-triene mit einem 21,16α-Laktonring nach Anspruch 1, worin R¹¹ aus der Gruppe der folgenden Seitenketten ausgewählt ist Hexyl, Octyl, Decyl, Dodecyl.

4. 11β-substituierte 19-Nor-17α-pregna-1,3,5(10)-triene mit einem 21,16α-Laktonring nach Anspruch 1, worin R³ ein Wasserstoffatom ist.

5. 11β-langkettig substituierte 19-Nor-17α-pregna-1,3,5(10)-triene mit einem 21,16α-Laktonring nach Anspruch 1, nämlich
3,17β-Dihydroxy-11β-hexyl-19-nor-17α-pregna-1,3,5(10)-trien-21,16α-lakton
3,17β-Dihydroxy-11β-octyl-19-nor-17α-pregna-1,3,5(10)-trien-21,16α-lakton
3,17β-Dihydroxy-11β-decyl-19-nor-17α-pregna-1,3,5(10)-trien-21,16α-lakton
3,17β-Dihydroxy-11β-dodecyl-19-nor-17α-pregna-1,3,5(10)-trien-21,16α-lakton

6. Pharmazeutische Präparate enthaltend mindestens eine Verbindung der allgemeinen Formel II gemäß Anspruch 1 sowie einen pharmazeutisch verträglichen Träger.

7. Verwendung der Verbindungen der allgemeinen Formel II gemäß Anspruch 1 zur Herstellung von Arzneimitteln.

8. Verwendung der Verbindungen der allgemeinen Formel II gemäß Anspruch 7 zur Herstellung von pharmazeutischen Zusammensetzungen bzw. Arzneimitteln zur Behandlung von estrogenabhängigen Krankheiten.

9. Verwendung der Verbindungen der allgemeinen Formel II gemäß Anspruch 7 zur Herstellung von pharmazeutischen Zusammensetzungen bzw. Arzneimitteln zur Behandlung von Mammakarzinomen, Endometriumskarzinomen und Prostatakarzinomen.

## Claims

1. 19-Nor-17α-pregna-1,3,5(10)-trienes with a 21,16α-lactone ring of general formula (II) 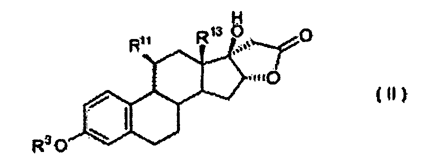 in which
R³ means a hydrogen atom, a C₁₋₄-alkyl, C₂₋₆-acyl or tri(C₁₋₄-alkyl)silyl group or a group R¹⁸SO₂-,
whereby
R¹⁸ means a group R¹⁹R²⁰N-,
in which
R¹⁹ and R²⁰, independently of one another, are a hydrogen atom, a C₁₋₅-alkyl radical, a group C(O)R²¹ and
in which
R²¹ means a straight-chain or branched hydrocarbon radical with up to
6 carbon atoms, a C₃₋₇-cycloalkyl radical, an aryl radical,
which optionally can be substituted, an aralkyl radical
or an alkylaryl radical,
and
R¹¹ means a straight-chain alkyl radical with 6 to 17 carbon atoms,
and
R¹³ means a methyl or ethyl group.

2. 11β-Substituted 19-nor-17α-pregna-1,3,5(10)-trienes with a 21,16α-lactone ring according to claim 1, **characterized in that** the compounds represent selective estrogen receptor α-antagonists.

3. 11β-Substituted 19-nor-17α-pregna-1,3,5(10)-trienes with a 21,16α-lactone ring according to claim 1, in which R¹¹ is selected from the group of the following side chains: hexyl, octyl, decyl and dodecyl.

4. 11β-Substituted 19-nor-17α-pregna-1,3,5(10)-trienes with a 21,16α-lactone ring according to claim 1, in which R³ is a hydrogen atom.

5. 11β-Long-chain-substituted 19-nor-17α-pregna-1,3,5(10)-trienes with a 21,16α-lactone ring according to claim 1, namely
3,17β-Dihydroxy-11β-hexyl-19-nor-17α-pregna-1,3,5 (10)-triene-21,16α-lactone
3 , 17β-Dihydroxy- 11β-octyl-19-nor-17α-pregna-1,3,5(10)-triene-21,16α-lactone
3,17β-Dihydroxy-11β-decyl-19-nor-17α-pregna-1,3, 5(10)-triene-21,16α-lactone
3 , 17β-Dihydroxy-11β-dodecyl-19-nor-17α-pregna-1,3,5(10)-triene-21,16α-lactone.

6. Pharmaceutical preparations that contain at least one compound of general formula II according to claim 1 as well as a pharmaceutically compatible vehicle.

7. Use of the compounds of general formula II according to claim 1 for the production of pharmaceutical agents.

8. Use of the compounds of general formula II according to claim 7 for the production of pharmaceutical compositions or pharmaceutical agents for treating estrogen-dependent diseases.

9. Use of the compounds of general formula II according to claim 7 for the production of pharmaceutical compositions or pharmaceutical agents for treating breast cancer, endometrial carcinoma and prostate cancer.

## Revendications

1. 19-Nor-17α-pregna-1,3,5(10)-triènes ayant un noyau 21,16α-lactone de formule générale (II) 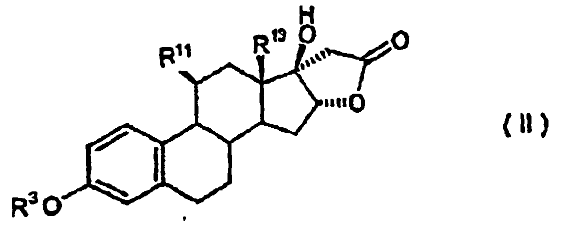 dans laquelle
R³ est un atome d'hydrogène, un groupe alkyle en C₁₋₄, acyle en C₂₋₆ ou tri (alkyle en C₁₋₄) silyle ou un groupe R¹⁸SO₂-,
où R¹⁸ est un groupe R¹⁹R²⁰N-, où R¹⁹ et R¹⁸ représentent chacun indépendamment de l'autre un atome d'hydrogène, un radical alkyle en C₁₋₅, un groupe C (O) R²¹, et où R²¹ représente un résidu hydrocarboné à chaîne droite ou ramifiée ayant jusqu'à 6 atomes de carbone, un radical cycloalkyle en C₃₋₇, un radical aryle pouvant être éventuellement substitué, un radical aralkyle ou un radical alkylaryle, et
R¹¹ est un radical alkyle à chaîne droite ayant 6 à 17 atomes de carbone, et
R¹³ est un groupe méthyle ou éthyle.

2. 19-Nor-17α-pregna-1,3,5(10)-triènes 11β-substitués ayant un noyau 21,16α-lactone selon la revendication 1, **caractérisés en ce que** les composés représentent des antagonistes sélectifs du récepteur α des oestrogènes.

3. 19-Nor-17α-pregna-1,3,5(10)-triènes 11β-substitués ayant un noyau 21,16α-lactone selon la revendication 1, dans lesquels R¹¹ est choisi dans le groupe des chaînes latérales suivantes : hexyle, octyle, décyle, dodécyle.

4. 19-Nor-17α-pregna-1,3,5(10)-triènes 11β-substitués ayant un noyau 21,16α-lactone selon la revendication 1, dans lesquels R³ est un atome d'hydrogène.

5. 19-Nor-17α-pregna-1,3,5(10)-triènes substitués à longue chaîne en 11β ayant un noyau 21,16α-lactone selon la revendication 1, à savoir la 3,17β-dihydroxy-11β-hexyl-19-nor-17α-pregna-1,3,5(10)triène-21,16α-lactone, la 13,17β-dihydroxy-11β-octyl-19-nor-17α-pregna-1,3,5(10)-triène-21,16α-lactone, la 3,17β-dihydroxy-11β-décyl-19-nor-17α-pregna-1,3,5(10)triène-21,16α-lactone, la 3,17β-dihydroxy-11β-dodécyl-19-nor-17α-pregna-1,3,5(10)triène-21,16α-lactone.

6. Préparations pharmaceutiques contenant au moins un composé de formule générale (II) selon la revendication 1, ainsi qu'un excipient acceptable d'un point de vue pharmaceutique.

7. Utilisation des composés de formule générale (II) selon la revendication 1 pour préparer des médicaments.

8. Utilisation des composés de formule générale (II) selon la revendication 7 pour préparer des compositions pharmaceutiques ou des médicaments destinés au traitement de maladies dépendant des oestrogènes.

9. Utilisation des composés de formule générale (II) selon la revendication 7 pour préparer des compositions pharmaceutiques ou des médicaments destinés au traitement des cancers du sein, des cancers de l'endomètre et des cancers de la prostate.
